# EUROPÄISCHE PATENTSCHRIFT

(11) **EP 0 646 261 B1**
(45) Veröffentlichungstag und Bekanntmachung des Hinweises auf die Patenterteilung: **29.01.1997**
(21) Anmeldenummer: 93913016.7
(22) Anmeldetag: 18.06.1993
(51) Int. Cl.: G06F 17/00

(54) **VERFAHREN UND EINRICHTUNG ZUR ANALYSE VON HOCHDYNAMISCHEN SEKRETIONSPHÄNOMENEN VON HORMONEN IN BIOLOGISCHEN DYNAMISCHEN SYSTEMEN MITTELS BIOSENSOREN**
METHOD AND DEVICE FOR THE ANALYSIS, USING BIOSENSORS, OF HIGHLY DYNAMIC HORMONE-SECRETION PHENOMENA IN DYNAMIC BIOLOGICAL SYSTEMS
PROCEDE ET DISPOSITIF VISANT A ANALYSER AU MOYEN DE BIODETECTEURS LES PHENOMENES HAUTEMENT DYNAMIQUES DE SECRETION D'HORMONES DANS DES SYSTEMES BIOLOGIQUES DYNAMIQUES

(30) Priorität: 19.06.1992 DE 4220169
(43) Veröffentlichungstag der Anmeldung: 05.04.1995
(73) Patentinhaber: HESCH, Rolf Dieter, Prof. Dr., D-78464 Konstanz (DE)
(72) Erfinder: HESCH, Rolf Dieter, Prof. Dr., D-78464 Konstanz (DE)
(74) Vertreter: Boeters, Hans Dietrich, Dr.
(86) Internationale Anmeldenummer: EP9301566
(87) Internationale Veröffentlichungsnummer: WO9400818

(56) Entgegenhaltungen:
- COMPUTERS & CHEMICAL ENGINEERING Bd. 16, Nr. 4 , April 1992 , UK Seiten 283 - 291 C. DI MASSIMO ET AL 'TOWARDS IMPROVED PENICILIN FERMENTATION VIA ARTIFICIAL NEURAL NETWORKS'
- IEEE TRANSACTIONS ON BIO-MEDICAL ENGINEERING Bd. 38, Nr. 2 , Februar 1991 , NEW YORK US Seiten 113 - 125 A.H.VAGNUCCI ET AL 'CLASSIFICATION OF PLASMA CORTISOL PATTERNS IN NORMAL SUBJECTS AND IN CUSHING'S SYNDROME'
- PROCEEDINGSOF THE FOURTEENTH ANNUAL NORTHEAST BIOENGINEERING CONFERENCE 10. März 1988 , DURHAM, NH, USA Seiten 257 - 300 XP000093492 T.P.WANG ET AL 'WIGNER-VILLE SPECTRUM ANALYSIS OF HORMONAL TIME SERIES'
- PROCEEDINGS OF THE ANNUAL CONFERENCE OF THE IEEE ENGINEERING IN MEDICINE AND BIOLOGY SOCIETY Bd. 13, Nr. 4 , 31. Oktober 1991 , ORLANDO, FLORIDA, USA Seiten 1660 - 1661 XP000348137 J.M.GOLDMAN 'NEURAL NETWORK ANALYSIS OF PHYSIOLOGIC WAVEFORMS'
- IEEE TRANSACTIONS ON BIO-MEDICAL ENGINEERING Bd. 38, Nr. 2 , Februar 1991 , NEW
- YORK US Seiten 113 - 125 A.H.VAGNUCCI ET AL 'CLASSIFICATION OF PLASMA CORTISOL PATTERNS IN NORMAL SUBJECTS AND IN CUSHING'S SYNDROME'

## Beschreibung

Die vorliegende Erfindung befaßt sich mit einem Verfahren und einer Einrichtung zur Analyse von hochdynamischen Sekretionsphänomenen von Hormonen in Blut-Zirkulationssystemen mittels Biosensoren, insbesondere mit einem Verfahren und einer dieses Verfahren anwendenden Einrichtung zur Erkennung von dynamischen Mustern (Code) und deren Abänderung bei dynamischen Prozessen in lebenden biologischen dynamischen Systemen mittels hcchvernetzter künstlicher Intelligenz.

In den vergangenen Jahren wurden mehrfach Versuche unternommen, durch gezielte Maßnahmen Konzentrationsstörungen einer bestimmten Substanz, die auf herkömmliche Art im Blut gemessen wurde, durch gezielte Zufuhr bestimmter Stoffe zu beseitigen. Genau diese Therapie wird im Falle eines Diabetikers durch Abgabe von Insulin in das Blut-Zirkulationssystem durchgeführt.

Bisher wurde davon ausgegangen, daß sowohl Steroid- als auch Peptid- und Proteinhormone im Blut in konstanter Konzentration zirkulieren. Der betreffende Hormonspiegel, so wurde angenommen, lag bei Gesunden in einem Bereich, der durch Messungen vieler "normaler" Systeme als Mittelwert mit einem Standardabweichungsbereich als Normal- oder Referenzbereich definiert wurde. Pathologische Abweichungen des hormonalen Systems wurden dadurch definiert, daß bei bestimmten Symptomen einer Erkrankung, die den Verdacht auf eine hormonal-bedingte Ursache weckten, die Messung der betreffenden Hormonkonzentration vom Normal- oder Referenzbereich abweichen mußte. Bei einer Abweichung nach unten spricht man von Unterfunktion, bei einer Abweichung nach oben von einer Überfunktion. Heilung ging neben einer Symptombesserung mit einer Rückkehr der Hormonkonzentrationen in den Normal/Referenzbereich einher.

Mit diesem Verständnis werden Zustände, nämlich "Gesundheit" und "Krankheit" als getrennte Entitäten beschrieben, die sozusagen durch ärztliche Diagnose oder gleichwertige Zustandsbeschreibungen voneinander abgeschieden und meßbar dargestellt werden.

Schon in dieser klassischen Definition von Krankheit als einem statistischen Ereignis, das einem einzelnen im Vergleich mit einer Population als Zustandsänderung widerfährt, wird deutlich, daß eine gewisse Zeitkomponente zu berücksichtigen ist, nämlich der Übergang von gesund zu krank. Dieser erfolgt einmal subjektiv im Erleben des Betroffenen als Patienten, das sich aber der kommunizierbaren Messung verschließt, und zum anderen sprunghaft während des Ereignisses der Feststellung einer meßbaren Abweichung und deren Offenbarung als Krankheit des Betroffenen. Diese statistische Medizin hat einen großen diagnostischen Fortschritt in der modernen Beschreibung von Krankheit als einer meßbaren Abweichung ermöglicht und bedeutet eine der größten Errungenschaften gegenwärtiger Medizin in allen Bereichen, wobei hier das Beispiel der Hormone als Paradigma steht.

Aus dem Stand der Technik sind aus dem Artikel von C. di Massimo et al. in Computers Chem. Engng. Vol. 16 No. 4, S. 283-291, 1992, Methoden bekannt, die einen technischen Regelvorgang, z.B. einen technischen Fermentationsprozeß mit statistischen Methoden analysieren und eine Klassifizierung der Meßgrößen dieses biologischen Systems mit künstlichen neuronalen Netzwerken vornehmen. Eine andere statistische Methode zur Analyse von Mustern ist aus dem Artikel von A.H. Vagnucci et al. in 8088 IEEE Transactions on Biomedical Engineering, Vol. 38 (1991) bekannt. Hier wird eine an sich schon lange bekannte statistische Analyse von Mustern beschrieben, die ebenfalls mit künstlichen neuronalen Netzwerken bei der Penicillin-Fermentation erstellt wird. Diese Methoden reichen jedoch nicht aus, um eine Analyse eines lebenden biologischen Systems vorzunehmen, in dem eine codierte Information enthalten ist.

Bei genauerer Betrachtung fällt jedoch unmittelbar auf, daß raum-zeitliche Krankheitsentwicklungen im Sinne einer "Krankheitsanbahnung" des Betroffenen aus einem Zustand des Gesundseins zu einem Zustand des Krankseins hierdurch nicht beschrieben werden können. Krankheit als Biographie war bislang nicht meßbar. Der Zeitraum zwischen gesund und krank ist nicht mit Laborwerten ausmeßbar und, wenn dies in der statistischen Medizin, die nie nur den einzelnen, sondern immer sein Verhalten im Vergleich zur Kontrollpopulation berücksichtigen muß, untersucht wurde, so fielen häufig pro Zeiteinheit große Datensätze an, die aus Mangel an Übersicht keinen Sinn ergaben oder einen solchen nur kurzfristig zu erkennen geben. Als Beispiel kann der Kranke auf der Intensivstation gelten, wo relativ zahlreiche, kurzfristig hintereinander gewonnene Laborwerte eine Definition des Zustands des Patienten ermöglichen soll. Es ist aber bekannt, daß nur etwa 10% der Werte überhaupt vom Krankheitsverständnis her zur Beurteilung des Zustandes des Kranken herangezogen werden, der Rest wird nicht verwertet und als stochastisches Rauschen bezeichnet, weil kein erkennbarer Sinn in den gesammelten Daten liegen soll.

Ein weiteres Beispiel ist der zeitliche Zusammenhang von Glucose und Insulin beim Normalen und beim Diabetiker. Bisher wurde über einen relativ großen Zeitraum von maximal 24 Stunden und weniger die Insulinabgabe dem Blutzucker angepaßt. Inzwischen weiß man aber, daß dieser Zusammenhang bei Normalen in vielen kleineren Zeitabschnitten besteht und daß der Diabetiker zum Beispiel am besten durch Insulinpumpen optimal zu versorgen ist.

Bei genauerer Betrachtung dieser Erkenntnis gelangt man zu einem neuen Krankheitsverständnis, welches sich in jüngster Zeit über dem bisher gültigen statistischen Konzept eines linearen Zusammenhangs von Ursache und Wirkung mit kausalen Zustandsänderungen (Gesundheit/Krankheit) entwickelt. In der Biologie wurden nach neueren Erkenntnissen aus der Physik Experimente zur zeitlichen Programmierung der biologischen Signalübertragung durchgeführt. Dabei hat sich gezeigt, daß biologische Signale, welche an den für die spezifischen Rezeptoren empfangen und in biologische Information umprogrammiert werden, bei dieser Informationsübertragung schnell an- und abgeschaltet werden. Ebenso schaltet der Rezeptor seine Empfangsbereitschaft in Abhängigkeit vom Signal und in Abhängigkeit von der Rezeptorsignalverarbeitung auf und ab (Rezeptorsensitivierung und -desensitivierung). Mit diesem dynamischen "Zusammenspiel" der Signalbereitstellung, Verarbeitung und Beendigung baut die Natur komplexe biologische Regelsysteme in Zell- aber auch auf Organebene nach dem Prinzip der Selbstorganisation auf.

Wenn man diese Erkenntnis der Biologie nun auf die Signalkodierung in Hormonen anwendet, bietet es sich an, daß das Verhalten der zirkulierenden Hormonkonzentrationen einmal nicht nach statisch-statistischen mit situativen Messungen an einem beliebigen Zeitpunkt zu untersuchen, um diese mit dem Normal-Referenzbereich zu vergleichen. Vielmehr empfiehlt es sich, ein enges Zeitraster, wie es aus der biologischen Signalübermittlung bekannt ist, zu analysieren. Die Messung von Hormonen im 10-, 5-, 2- und 1-Minutentakt hat nun ergeben, daß die meisten Hormone im Blut nicht konstant zirkulieren, sondern einer intensiven Pulsamplituden- und -frequenzmodulation unterliegen, d.h. die Hormonkonzentration schwankt in Raum und Zeit. Es lassen sich raum-zeitliche Hormonmuster beschreiben, die für ein Individuum typisch sein können. In den letzten Jahren sind auch raum-zeitliche Muster beschrieben worden, die bestimmten Krankheiten zugeordnet sind. Dabei fällt auf, daß sich solche dynamischen Hormonmuster beim Normalen im bekannten "Normal/Referenzbereich" bewegen, maximale und minimale Ausschläge aber auch über oder unter der Standardabweichung liegen können.

Es fällt ferner auf, daß gestörte Muster bei Krankheit durchaus ebenfalls im "Normal/Frequenzbereich" zu liegen kommen. Der Übergang von gesund zu krank entwickelt sich also dynamisch im Normalbereich biologischer Signalübertragung bei jedem einzelnen Individuum. Es wird von "dynamischer Krankheit" gesprochen und beobachtet nicht mehr Zustandsänderung des Einzelnen im Vergleich zum Kollektiv, sondern man beschreibt hier dynamische Hormonmusteränderungen innerhalb einer "biographischen Medizin" am einzelnen Individuum.

Dieses neue Konzept stellt damit eine Fortentwicklung der statistischen Medizin auf eine evolutionär höhere Stufe dar. Es gestattet, die raum-zeitliche Entwicklung von krankhaften Veränderungen am einzelnen Individuum an sich zu beobachten. Ein ideales Verfahren wäre, solche gestörten dynamischen Informationsübertragungen, wenigstens in Grenzen, durch Rekonstruktion der zerstörten Hormonmuster wiederherzustellen, was in Ansätzen z.B. durch die Insulinpumpentherapie verfolgt wird, ohne daß hierbei eine deterministische Codierung verwendet wird. Hierbei handelt es sich um eine sogenannte Trendanalyse, bei der aus statistischen Daten der Vergangenheit eine operationale Prognose in die Zukunft gemacht wird, unter der Voraussetzung, daß sich die Zukunft statistisch, d.h. zufällig und linear nach Ursache und Wirkung wie die Statistik der Vergangenheit entwickelt. Dies ist eine deduktive Methode, welche Ergebnisse der Vergangenheit als Erwartung in die Zukunft projiziert.

Dieses Vorgehen ist praktisch überall in der Regeltechnik Biologie und den prognostizierenden Wissenschaften nicht hinreichend erfolgreich gewesen, weil die Wirkkräfte der uns umgebenden Welt nicht linear geordnet sind.

Es ist ein Anliegen zukünftiger Medizintechnologie, Hormonmuster, aber auch raum-zeitliche Muster von Substanzen, z.B. Glucose, Elektrolyte, Blutgase, im Rahmen von Krankheitsbeschreibungen und deren Therapie kontinuierlich meßbar zu machen. Dies ist durch die bisherige Technologie der häufigen Blutentnahme nicht mehr darstellbar.

Hierzu müssen also Sensoren entwickelt werden, welche über längere Zeit in der Blutbahn verweilen können, um raum-zeitliche Hormonmusteranalysen aufzunehmen.

Die bisherigen klassischen statistischen Analysen der Datenfülle erlauben lediglich die statistische Zuordnung der Richtigkeit der Meßwerte, sie gestatten aber keine Zuordnung einer Bedeutung, eines "Sinnes" in den Daten. Aus der synergetischen Cooperation der Funktion dynamischer Systeme (Chaoslehre) ist bekannt, undurchsichtige komplexe Vorgänge zu beschreiben. So ist es beispielsweise möglich, zu erkennen, daß aus der Fülle der Daten von dynamischen Hormonanalysen nicht eine Zufälligkeit steckt, sondern daß dahinter ein Determinismus, ein Programm bzw. ein Code verborgen ist, den wir mit den mathematischen Gesetzmäßigkeiten des deterministischen Chaos erfassen können.

Damit hat ein raum-zeitliches Muster eines Menschen eine für ihn typische Bedeutung, die mit seinem Gesundsein direkt zusammenhängt. Eine Zerstörung der raum-zeitlichen Hormonmuster in der biologischen Informationsübertragung ist mit Krankheit verbunden. Diagnostik am Individuum wird also die Beschreibung seiner Informationsmuster und deren Zerstörung durch Krankheit sein, und nicht mehr der statistische Vergleich mit Normal/Referenzbereichen einer Population, d.h. also nicht mehr zwei Zustände (gesund/krank), sondern eine individuelle Musteranalyse beschreibt den Zustand des Individuums. Die Überlegungen gehen davon aus, daß die erhaltenen Meßdaten zu zirkulierenden Hormonkonzentrationen in ihrer Pulsamplituden- und Frequenzmodulation nicht stochastisch-statistisch, also zufällig, geordnet sind. Im Gegensatz dazu benutzen Trendanalysen, wie bereits oben erwähnt, stochastisch-statistische Daten aus vergangenen Messungen und ordnen diese statistisch, um daraus sinnvolle und logische Erwartungen für die Zukunft zu erstellen. Bei diesen Schätzungen der Trendanalysen kann allerdings der systemimmanente Zufall die Annahmen, wie die Erfahrung zeigt, erheblich falsifizieren.

Grundvoraussetzung für die vorliegende Erfindung ist es also, daß nicht der Zufall, sondern ein mathematisch berechenbarer Determinismus, also ein Code in den Meßdaten verborgen liegt. Dieser Code wird im folgenden, im Gegensatz zum genetischen Code, als dynamischer Code bezeichnet. Dabei wird stets davon ausgegangen, daß biologische Regelvorgänge an Zellen, Organen und dem Gesamtorganismus zum Beispiel durch Hormone in deterministischen Zeitreihen organisiert sind. Hierdurch werden in einer hohen Ordnung, die bislang nicht erkennbar war, alle zellulären Vorgänge des Organismus geregelt und kontrolliert. Eine derartige Codierung ordnet sich nach den Gesetzen der Selbstorganisation zu einem Determinismus. Der biologische Determinismus des dynamischen Code, der im Gegensatz zum Zufall den Sinn meßbar macht, erlaubt nun erstmalig ein "Lernen aus der Zukunft".

Als unerläßliches Element zur Analyse von Daten eines implantierten Biosensors wird also ein computergestützes Verfahren hochvernetzter künstlicher Intelligenz benötigt, welches Muster und Strukturen, wie sie in raum-zeitlichen Hormonzeitreihen gefunden wurden, erkennt und sinngemäß zuordnen kann.

Für diesen Zweck bieten sich sogenannte Neurocomputer oder neuerdings neuronale Netzwerke an. Aber jede andere Form von Expertensystemen, die Unschärfekalküle (Fuzzy-Logic) durchführen können, sind geeignet, Klassifizierungen von deterministischen Mustern durchzuführen.

Werden nun Meßreihen von Parathormonen einem neuronalen Netzwerk eingespeichert, so kann das Netzwerk den Determinismus trainieren und erlernen. Aus dem Gelernten kann das neuronale Netzwerk in der Gegenwart die Meßwerte, die in Zukunft gemessen werden, für eine gewisse Zeit, bei Parathormon bis zu 28 Minuten, voraussagen. Diese Voraussagung wird immer nur begrenzt sein, da alle biologischen und evolutiven Systeme aufgrund der Unschärfe der initialen Randbedingungen ihres Ablaufs extrem sensitiv auf diese reagieren. Ein Determinismus in der Evolution ist stets endlich limitiert, jedoch kann diese Endlichkeit mit neuronalen Netzwerken in ihrem Muster gelernt und erkannt werden.

Diese Erkenntnis intensiver Untersuchungen führt nun dazu, Kranksein neu zu klassifizieren und daraus therapeutische Rückschlüsse zu ziehen.

Daher ist es Aufgabe der vorliegenden Erfindung, ein Verfahren bzw. eine dieses Verfahren durchführende Dosiereinrichtung bereitzustellen, die in der Lage sind, aus kontinuierlich aufgenommenen Daten eines lebenden biologischen Systems ein raum-zeitliches Muster (Code) der Daten zu ermitteln, um Informationen über chemische Veränderungen zwischen Gesundheit und Krankheit zu erhalten, um rechtzeitig eine daraus ableitbare Medikamenten dosierung vorzunehmen.

Die oben gestellte Aufgabe der vorliegenden Erfindung wird ausgehend vom Oberbegriff der Ansprüche 1 und 9 mit den kennzeichnenden Merkmalen dieser Ansprüche gelöst.

Das erfindungsgemäße computergestützte Verfahren mit hochvernetzter künstlicher Intelligenz zur Analyse eines biologischen dynamischen Systems, das sich nach den Gesetzmäßigkeiten der Selbstorganisation verhält, geht von folgenden bekannten Verfahrensschritten aus:
- Speichern von kontinuierlichen Meßreihen von verschiedenen Meßgrößen eines bekannten biologischen Systems, die für einen bestimmten Krankheitsfall symptomatisch sind;
- Trainieren eines künstlichen neuronalen Netzes, das mit einem Datenverarbeitungsprogramm simuliert wird, mit Hilfe der von dem bekannten biologischen System aufgenommenen Meßreihen;
- Aufnahme von kontinuierlichen Meßreihen, die für einen bestimmten Krankheitsfall symptomatisch sind, mittels mindestens eines Biosensors, der im biologischen System implantiert ist, und dessen Meßsignale dem neuronalen Netzwerk zugeführt werden;
und ist durch die folgenden weiteren Verfahrensschritte gekennzeichnet:
- Analysieren der Änderungen der von dem künstlichen neuronalen Netzwerk erstellten raum-zeitlichen Muster der verschiedenen Meßgrößen;
- Vorausbestimmung des zukünftigen Verlaufs der Meßgrößen mit Hilfe dieser Analyse; und
- Einstellen des Stellgliedes einer Therapieeinrichtung anhand der vorausbestimmten Werte der Meßgrößen.

Aus dieser Voraus- oder "Wahrmessung" der Zukunft, was nicht gleichbedeutend ist mit einer Voraussage, Schätzung oder Trendanalyse, kann man nun prospektiv für die Gegenwart lernen. Wenn die in Zukunft eintretenden Ereignisse bei unveränderter Parameterkonstellation der Gegenwart nicht wünschenswert sind, so müssen die Randbedingungen der nichtlinearen dynamischen Systementwicklung neu eingestellt werden. Es wird also nicht ein Trend analysiert, sondern es wird aus der Zukunft, welche in die Gegenwart durch Meßdaten projiziert worden ist, gelernt.

Es erweist sich ferner bei der Erfindung als vorteilhaft, Muster der Zukunft mit bekannten Mustern zu vergleichen, um eine Auskunft darüber zu erhalten, ob mit pathologischen Abweichungen vom gewünschten Determinismus zu rechnen ist. Dies ist selbstverständlich prinzipiell nicht mit der statistischen Normwertkontrolle zu vergleichen, zum Beispiel ob der Blutzucker hoch oder niedrig ist, sondern es werden dynamische Muster analysiert und ein dynamischer Code erstellt. Diese können sich im Normwertbereich entwickeln und sich pathologisch verändern, was zur Krankheit führen kann, wie das Beispiel der oben erwähnten Osteoporose gezeigt hat.

Die erfindungsgemäße Einrichtung, die das oben genannte Verfahren anwendet, besteht aus
- mindestens einem Biosensor, der in einem biologischen System implantiert ist und für einen bestimmten Krankheitsfall symptomatische Meßsignale einem trainierten neuronalen Netz zuführt; und
- einem Datenverarbeitungsprogramm zur Simulation des neuronalen Netzes, das aufgrund der Signale raum-zeitliche Muster der verschiedenen, für einen bestimmten Krankheitsfall symptomatischen Meßgrößen erstellt; und ist dadurch gekennzeichnet, daß
- das Datenverarbeitungsprogramm die Änderungen der raum-zeitlichen Muster analysiert und den zukünftigen Verlauf der Meßgrößen mit Hilfe dieser Analyse vorausbestimmt; und
- Stellglieder anhand der vorausbestimmten Werte der Meßgrößen eingestellt werden.

Bei dem erfindungsgemäßen Verfahren erweist es sich als besonders vorteilhaft, in einem lebenden biologischen dynamischen System mindestens zwei Biosensoren zu implementieren, die kontinuierlich Signale einem Datenverarbeitungs-System zuführen. Dieses Datenverarbeitungs-System muß daher in der Lage sein, eine große Menge von Daten in kurzer Zeit zu erfassen und zu verarbeiten. Hierzu eignen sich insbesondere sogenannte Neurocomputer bzw. neuronale Netzwerke, die zur Berechnung der genannten Muster auch Fuzzy-Logic-Methoden verwenden. Aus den erkannten und berechneten bestimmten dynamischen raum-zeitlichen Mustern können dann vorteilhafterweise bestimmte Krankheitszustände entsprechenden Mustern zugeordnet werden.

Um erfindungsgemäß ein individuelles gestörtes lebendes biologisches System in den ungestörten Zustand zurückzuführen, werden zweckmäßigerweise die erkannten Muster herangezogen, um die notwendigen Schritte einzuleiten.

Die erfindungsgemäße Einrichtung zur Analyse von hochdynamischen Sekretionsphänomenen von Hormonen in Blut-Zirkulationssystemen verwendet vorteilhafterweise Biosensoren, die über eine längere Zeit in den lebenden dynamischen biologischen Systemen verweilen. Diese speziellen Biosensoren messen Substanzen, insbesondere Hormone, in einem bestimmten Medium und geben vorteilhafterweise optische bzw. elektrische Signale an ein Datenverarbeitungs-System, das Unschärfeberechnungen (Fuzzy Logic) durchführt, ab. Dabei hat sich herausgestellt, daß die Verwendung von Hormonrezeptorproteinen zur Konstruktion von hormonmessenden Biosensoren besonders vorteilhaft sind, wobei es sich vorzugsweise um die Verwendung der sieben-fach membrangängigen Rezeptorproteine handelt, welche mit einem hydrophoben Proteinanteil in der Zellmembran aufgehängt sind und welche Informationen durch einen in das Zellinnere hineinragenden Peptidschwanz unterschiedlicher Länge vermitteln. Die erhaltene Information kann bekannten Transducern zugeführt werden. Als äußerst vorteilhaft zur kontinuierlichen Messung der Konzentration von Nebenschilddrüsenhormonen im zirkulierenden Blut hat sich als geeignetes Sensormolekül das gentechnologisch synthetisierte Protein des Nebenschilddrüsenhormonrezeptors erwiesen. Dieses sieben-fach membrangängige Rezeptorprotein wurde erst kürzlich sequenziert und kloniert. Auch sind Modifikationen der Proteinsequenz dieses Rezeptorproteins zu verwenden. Auch bieten sich als Transducer ebenfalls bereits bekannte Technologien an.

Die Kopplung der verwendeten Biosensoren an das Datenverarbeitungs-System kann sowohl optisch als auch elektrisch vorgenommen werden.

Weitere Merkmale der Erfindung ergeben sich aus den Unteransprüchen.

Anhand der Zeichnungen wird nunmehr die vorliegende Erfindung im einzelnen näher erläutert. Es zeigen:
- Fig. 1: ein schematisches Blockschaltbild der vorliegenden Erfindung;
- Fig. 2: einen repräsentativen Rhythmus der Konzentrationen des PTH-Hormons als Funktion der Zeit;
- Fig. 3: den Rhythmus der PTH-Hormonkonzentration im Blut eines Mannes mit Osteoporose als Funktion der Zeit;
- Fig. 4: die PTH-Konzentration im Plasma als Funktion der Zeit von gesunden Patienten (durchgezogene Linie) und Patienten mit Osteoporose (unterbrochene Linien);
- Fig. 5: Spektren der Fehlervoraussage als Funktion der Zeit aus Daten der Fig. 1 mit gesundem (A) und mit an Osteoporose erkrankten Patienten (B);
- Fig. 6: zwei Meßreihen, wobei die obere Meßreihe A die gemessene ist und die untere Meßreihe B die aufgrund des "Trainings" erstellte Meßreihe darstellt;
- Fig. 7: zwei Kurven, von denen Kurve C aufgrund von Zufallsdaten erstellt wurde und Kurve D aufgrund von PTH-Daten;
- Fig. 8: die Topologie der Rezeptorstrukturen in Membranen.

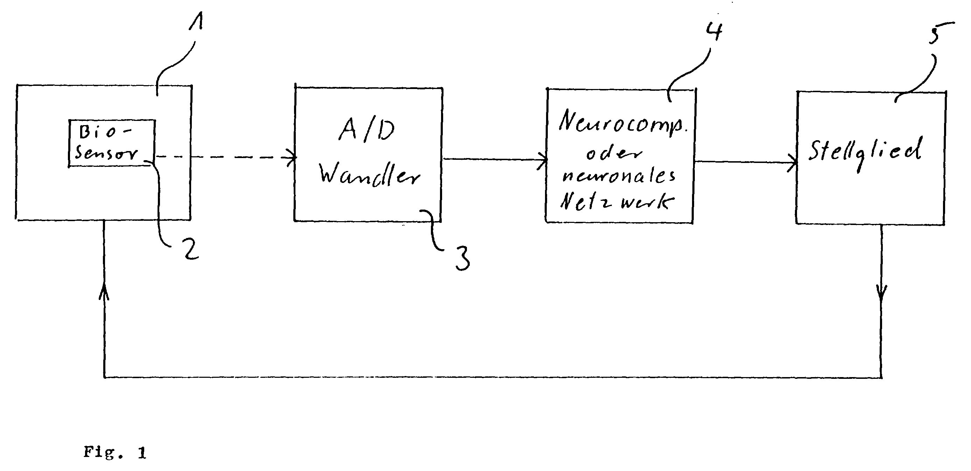

In Fig. 1 ist das Blockschaltbild einer erfindungsgemäßen Einrichtung zur Durchführung des erfindungsgemäßen Verfahrens schematisch dargestellt. Mit 1 ist das Blut-Zirkulationssystem gekennzeichnet, in dem ein Biosensor 2 implantiert ist und kontinuierlich analoge Signale einem A/D-Wandler 3 zuführt. Die Kopplung zwischen dem Biosensor 2 und dem A/D-Wandler 3 kann sowohl optisch als auch elektrisch vorgenommen werden. Der A/D-Wandler 3 liefert dann digitale Signale an ein Datenverarbeitungs-System, das sowohl einen Neurocomputer als auch ein neuronales Netzwerk beinhalten kann. Andererseits ist jedoch jede andere Form von Expertensystemen, die Unschärfekalküle (Fuzzy Logic) durchführen können, durchaus geeignet, Klassifizierungen von deterministischen Mustern durchzuführen. Die erhaltenen deterministischen Muster dienen, wie bereits oben erwähnt, dazu, das Kranksein zu klassifizieren und daraus therapeutische Rückschlüsse zu ziehen. Aus den Berechnungen der Neurocomputer bzw. der neuronalen Netzwerke 4 werden entsprechende Signale erzeugt, die einem Stellglied, z.B. einer computergesteuerten Applikationspumpe, zugeführt werden. Das Stellglied 5 bzw. die Therapieeinrichtung arbeitet dann in der Weise, daß sie versucht, das erkannte gestörte deterministische Muster in ein ungestörtes deterministisches Muster zurückzuführen.

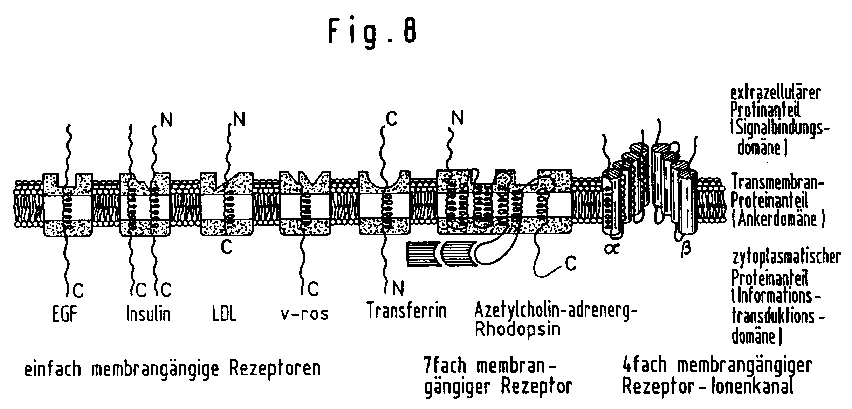

Ein entscheidender Punkt in dieser Anlage ist der Biosensor 2, der im Blut-Zirkulationssystem eines Individuums 1 implantiert ist. Ein brauchbarer denkbarer Biosensor muß die Eigenschaft haben, über längere Zeit in der Blutbahn weilen zu können, ohne schädliche Auswirkungen zu verursachen. Eine mögliche Lösung dieses Problems könnte ein Biosensor sein, der auf der Basis der in Fig. 8 dargestellten Konstruktionen membrangängiger Rezeptoren dargestellt ist.

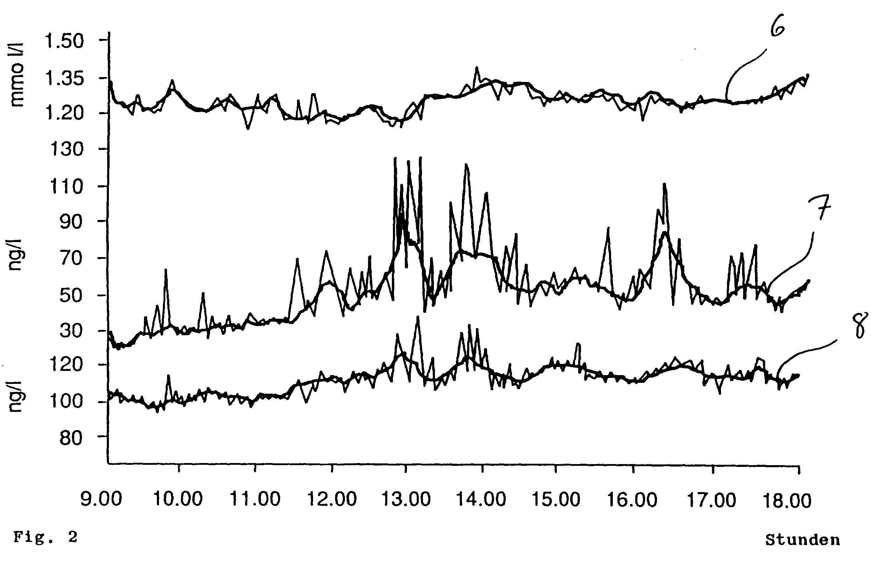

In Fig. 2 ist ein repräsentativer Rhythmus der PTH-Konzentration im Blut eines gesunden jungen Mannes dargestellt, wobei die obere Kurve 6 ionisiertes Calcium, die beiden unteren Kurven 7, 8 PTH-Konzentrationen darstellen, wobei ein 20-Minuten-Durchschnittswert für jede Kurve zusätzlich gezeichnet wurde, der durch die durchgezogene Linie dargestellt ist. Die Kurven zeigen, daß in einem normalen gesunden Individuum das PTH (Hormon) im Blut in pulsierender Weise zirkuliert. Die Konzentration eines intakten PTH (Hormons) innerhalb des Zirkulationssystems beträgt ungefähr 20 bis 40 pg/ml und pulsiert mit einer Höhe von etwa 200 pg/ml, wobei derartige Pulse häufig zu verzeichnen sind. Dieses Ergebnis zeigt, daß die Peaks fast zehnmal größer sind. Wichtig in dieser Darstellung ist es, zu erkennen, daß kräftige Konzentrationsschwankungen mit einem auf den ersten Blick irregulären Muster als Funktion der Zeit auftreten. Diese Muster sind mit komplizierten mathematischen Methoden zu analysieren.

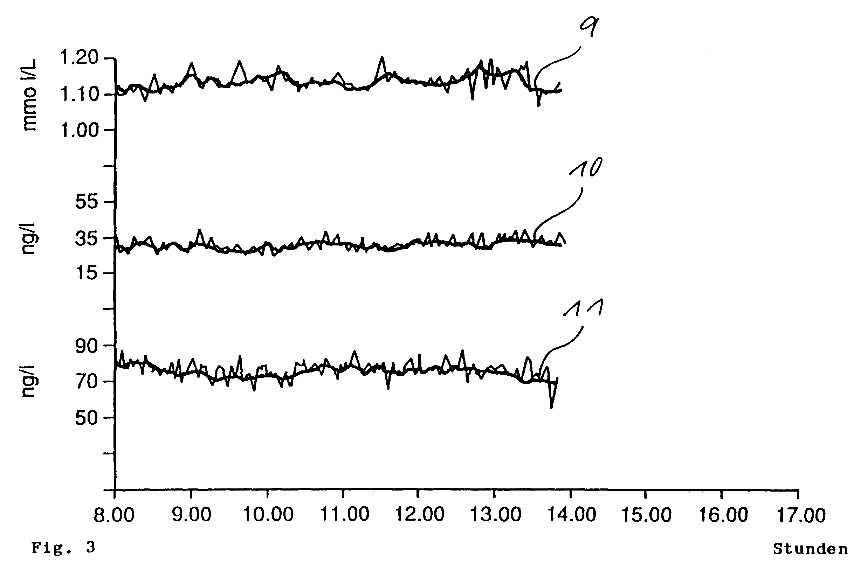

In Fig. 3 ist der Rhythmus der PTH-Konzentrationen eines männlichen Patienten mit Osteoporose als Funktion der Zeit dargestellt. In der oberen Kurve 9 ist ionisiertes Calcium in den unteren Kurven 10, 11 die PTH-Konzentration dargestellt. Hierin ist sehr deutlich eine Verringerung der Amplituden und Abnahme der Frequenz der PTH-Sekretion und damit der Konzentration sichtbar, was typisch ist für ein an Osteoporose erkranktes Individuum.

Bei näherer Betrachtung der beiden Diagramme in Fig. 2 und 3 ist also eine deutliche Änderung sowohl in der Frequenz als auch in der Amplitude der zeitabhängigen Spektren zu beobachten. Mit anderen Worten heißt das, daß ein gewisses Muster für den gesunden bzw. den kranken Zustand eines Individuums charakteristisch ist. Daher lassen sich hieraus therapeutische Maßnahmen herleiten, die das gestörte Muster des kranken Patienten wieder regenerieren.

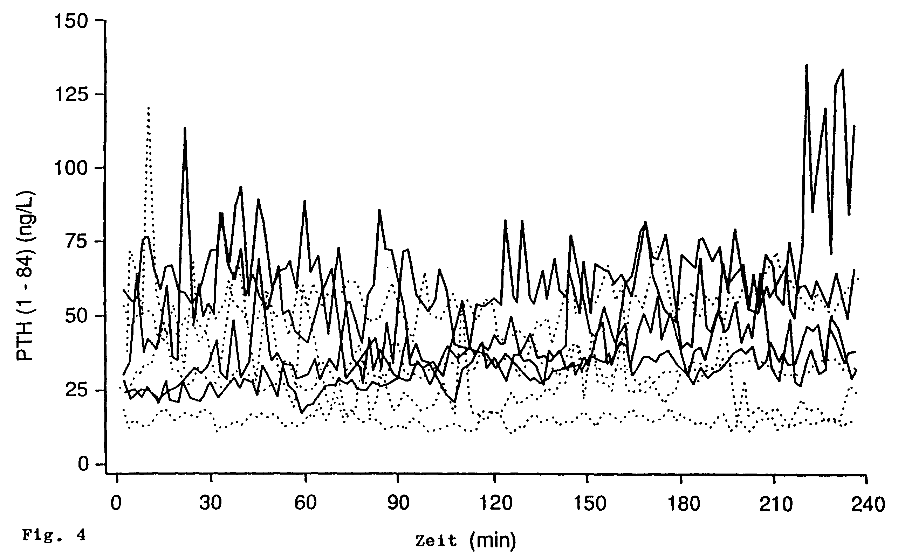

In Fig. 4 werden zeitabhängige Spektren der PTH-Konzentrationen in Plasmen während einer Periode von 4 Stunden dargestellt. Die durchgezogenen Linien geben die Ergebnisse eines gesunden Individuums wieder, während die unterbrochenen Linien Meßergebnisse von Patienten mit Osteoporose sind. Im folgenden werden kurz die untersuchten Individuen und Untersuchungsmethoden erläutert. Die untersuchten Individuen wurden in zwei Gruppen eingeteilt, wobei die erste Gruppe zwischen 24 und 26 Jahre alt war und gesund war. Die zweite Gruppe war zwischen 31 und 42 Jahre alt und an ideopatischer Osteoporose erkrankt. Die physischen Parameter der untersuchten Personen waren in beiden Gruppen normal. Die an Osteoporose erkrankten Patienten hatten jeweils zwei Rückgratfrakturen und eine reduzierte Knochenmasse. Die an Osteoporose erkrankten Personen zeigten ferner einen auffallenden Abfall der Knochenbildungsoberflächen. Um die kurzzeitigen Fluktuationen der PTH-Serumkonzentration zu untersuchen, wurden für die erste Gruppe 9 Stunden lang gemessen und für die zweite Gruppe 4 bis 6 Stunden lang. In beiden Gruppen wurden alle 2 Minuten Blutuntersuchungen über ein zentrales Venenkatheder durchgeführt.

Beide untersuchten Gruppen zeigen Bereiche, die quasi-periodische oder chaotisches dynamisches Verhalten zeigen. Dennoch können systematische Unterschiede im dynamischen Verhalten der Spektren gewonnen werden. Die Technik zur Analyse einer Fülle derartiger Daten erfordert Computer großer Kapazität, die den Anfall von derartig hohen Datenraten verarbeiten können. Ohne hier die Methode zur Analyse der in Fig. 4 gezeigten Daten im einzelnen aufzuführen, ist zu sagen, daß es gelungen ist, eine Klassifizierung einer allgemeinen deterministischen Komponente herauszufinden, die für das gesunde Individuum charakteristisch ist. Diese Komponente ist jedoch beinahe völlig ausgeblieben für den Fall eines an Osteoporose erkrankten Patienten, wie dies in Fig. 4 in den unterbrochenen Linien zu sehen ist.

Man kann nun ein neuronales Netzwerk, in diesem Falle eines mit 15 Eingängen und einem Ausgang benutzen, um es mit Meßwerten von Normalwerten zu trainieren. Die trainierten raum-zeitlichen Muster vergleicht das Netzwerk anschließend mit ähnlichen von Gesunden, und es soll erkennen können, welche von Kranken stammen. als Kriterium wird die Vorhersehbarkeit von Mustern in die Zukunft herangezogen, wobei die Höhe des Vorhersagefehlers (prediction error) bedeutend ist.

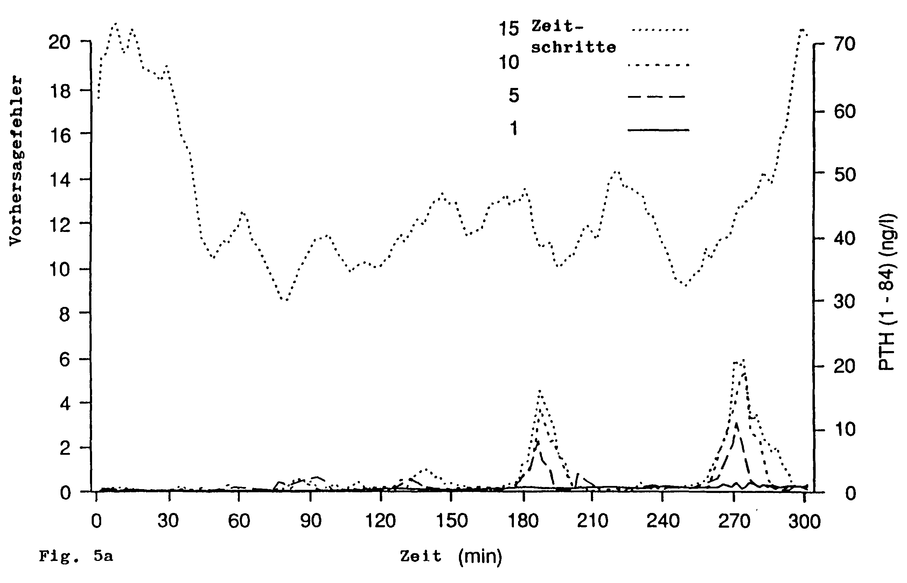

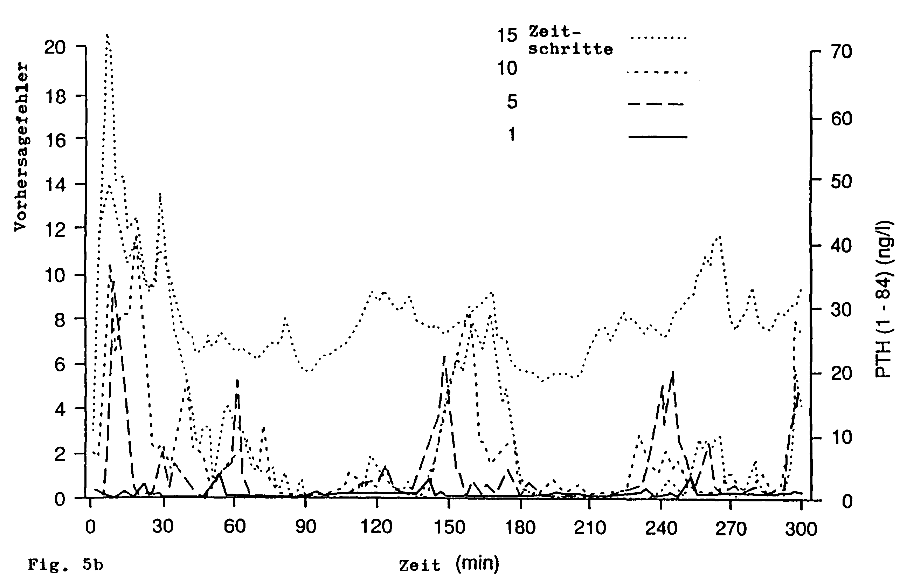

In Fig. 5 wird die Divergenz zwischen den vorhergesagten und den tatsächlich gemessenen zeitabhängigen Spektren für ein gesundes (A) bzw. erkranktes (B) Individuum dargestellt. Obwohl die vorausgesagten und beobachteten Fehlerspektren der PTH-Serumkonzentration im gesunden Individuum in einigen Sektionen signifikant divergieren, bleiben die meisten Teile der Spektren sehr nahe an den beobachteten Werten. Dabei werden als Parameter vorhergesagte Werte in eine Zukunft von 1, 5, 10 und 15 Zeitschritten gewählt. Je weiter in die Zukunft vorhergesagt werden kann, umso sicherer erkennt das Netzwerk typische raum-zeitliche Muster.

Es läßt sich nun zeigen, daß die Muster von Osteoporosekranken dadurch erkannt werden können, daß sie eine deterministische Ordnung, welche Vorhersagen erlaubt, verloren haben. Man kann also Krankheit als mangelnde gegenwärtige und zukünftige Ordnung messen, klassifizieren und zuordnen.

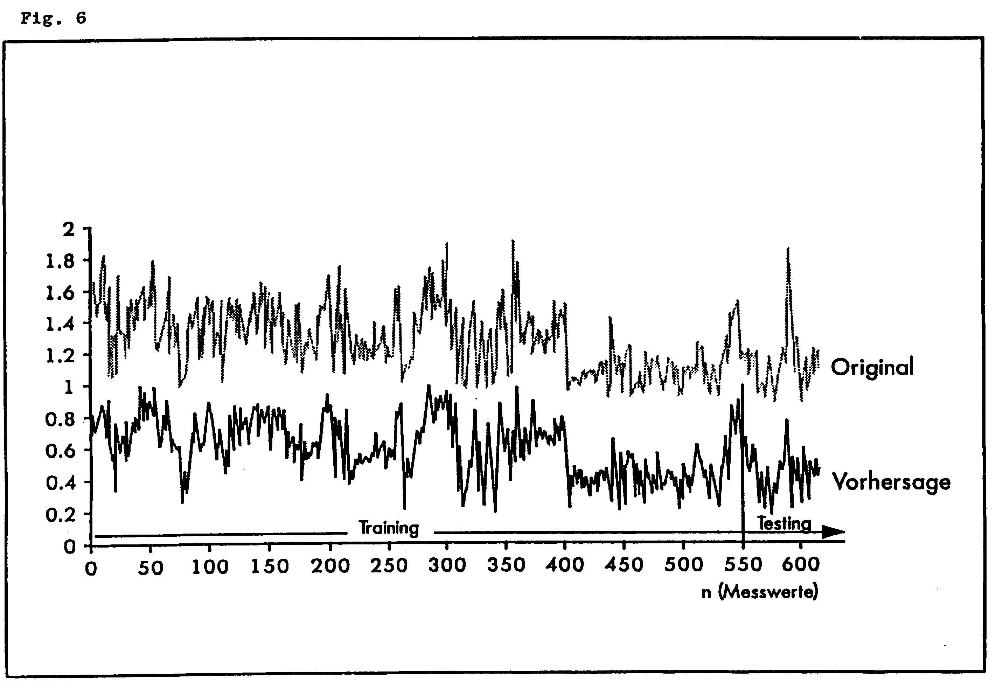

Fig. 6 zeigt zwei Meßreihen, wobei die obere Meßreihe A die gemessene ist und die untere Meßreihe B diejenige, die aufgrund des Trainings erstellt wurde. Das verwendete neuronale Netzwerk wurde mit einem PTH-Zeitmuster eines gesunden Systems mit einem limitierten Determinismus eine bestimmte Zeit lang trainiert. Die Abszisse dieser Darstellung ist die Zeitachse und enthält willkürliche Zahlen bei n = 70 Meßwerten, die alle zwei Minuten, also über einen Zeitraum von 35 Minuten gemessen und trainiert wurden. Diese Abbildung zeigt, daß ein neuronales Netzwerk 70 Meßwerte der Zukunft korrekt voraussagen kann, wenn man von systembedingten Schwankungen absieht. Die "Zukunft" ist also in den Meßdaten codiert oder programmiert; sie ist kein "Trend", sondern ein mathematisches Programm, das implizit in den Meßdaten zutage tritt. Man kann also aus der vorausgesagten Zukunft für die Gestaltung des biologischen dynamischen Systems der Gegenwart und für die Zukunft lernen.

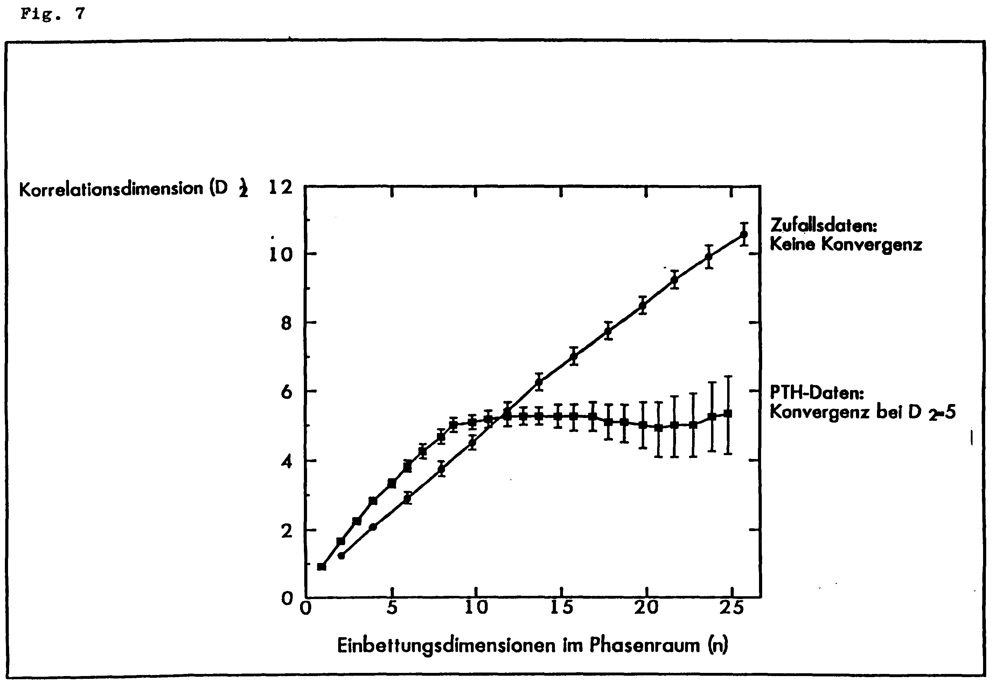

Fig. 7 zeigt zwei Kurven, von denen die Kurve C aufgrund von Zufallsdaten erstellt wurde und die Kurve D aufgrund von PTH-Daten. Die Abszisse dieser Darstellung enthält Zufallszahlen im Phasenraum, und die Ordinate gibt die Korrelation der Meßdaten wieder. Die Korrelation der Zufallsdaten in Kurve C verläuft linear ohne Konvergenz, wogegen die Meßdaten eines determinierten Chaos (Kurve D), wie es beispielsweise in einem biologischen dynamischen System vorzufinden ist, sich einem bestimmten Grenzwert nähern. Hieraus ist eindeutig zu erkennen, daß die Korrelation von stochastischen Zufallsdaten grundsätzlich unterschiedlich von der Korrelation von PTH-Daten ist. Daher ist das erfindungsgemäße Verfahren zur Bestimmung eines dynamischen Code eines biologischen dynamischen Systems nicht zu vergleichen mit einer linearen Extrapolation von Meßwerten, um daraus mögliche Meßwerte für die Zukunft zu erhalten.

In Fig. 8 wird die Topologie einiger an sich bekannter Strukturen eines Rezeptors in einer Membran für verschiedene membrangängige Rezeptoren dargestellt. Hierbei ist E das Effektor-Protein, G das G-Protein, C der C-Terminus, EGF der epiderme Wachstumsfaktor (epidermal growth factor), LDL die niedrige Dichte des Lipoproteins v-ros der viral-ros-Rezeptor. Auf der linken Seite der Darstellung sind einfache mehrmembrangängige Rezeptoren dargestellt, und in der Mitte mehrfach membrangängige Rezeptoren wie beispielsweise der sieben-fach membrangängige Rezeptor, der eine denkbare Lösung für den zu verwendenden Biosensor 2 darstellt.

Zusammenfassend ist festzustellen, daß mit den Ausführungen zur vorliegenden Erfindung ein Verfahren und eine Einrichtung zur Durchführung des erfindungsgemäßen Verfahrens vorgestellt wurde, die in der Lage sind, eine Analyse von hochdynamischen Sekretionsphänomenen von Hormonen in zirkulierenden Blut-Systemen durchzuführen. Die Ergebnisse des erfindungsgemäßen Verfahrens wurden am Beispiel der Osteoporose überzeugend dargestellt, so daß davon ausgegangen werden kann, daß mit einer erfindungsgemäßen dynamischen Diagnoseeinrichtung es in Zukunft möglich sein wird, den Vorgang des "Unterwegsseins" zwischen Gesundheit und Krankheit neu zu beschreiben, und therapeutische Maßnahmen aufgrund der Erkenntnisse zu ergreifen.

## Patentansprüche

1. Automatisches Dosierverfahren, bestehend aus den Verfahrensschritten:
- Speichern von kontinuierlichen Meßreihen von verschiedenen Meßgrößen eines bekannten biologischen Systems, die für einen bestimmten Krankheitsfalls symptomatisch sind;
- Trainieren eines künstlichen neuronalen Netzes (4), das mit einem Datenverarbeitungsprogramm simuliert wird, mit Hilfe der von dem bekannten biologischen System vorgenommenen Meßreihen;
- Aufnahme von kontinuierlichen Meßreihen, die für einen bestimmten Krankheitsfalls symptomatisch sind, mittels mindestens eines Biosensors (2), der im biologischen System implantiert ist und dessen Meßsignale dem neuronalen Netz zugeführt werden;
und **gekennzeichnet durch** die folgenden weiteren Verfahrensschritte:
- Analysieren der Änderungen der von dem künstlichen neuronalen Netzwerk (4) erstellten raum-zeitlichen Muster der verschiedenen Meßgrößen;
- Vorausbestimmung des zukünftigen Verlaufs der Meßgrößen mit Hilfe dieser Analyse; und
- Einstellen des Stellgliedes (5) einer Dosiereinrichtung anhand der vorausbestimmten Werte der Meßgrößen.

2. Verfahren nach Anspruch 1, **dadurch gekennzeichnet**, daß die erstellten raum-zeitlichen Muster bestimmten Krankheitszuständen zugeordnet werden.

3. Verfahren nach Anspruch 1, **dadurch gekennzeichnet**, daß jedem individuellen lebenden biologischen System eine individuelle Musteranalyse, die den Zustand des biologischen Systems beschreibt, zugeordnet wird.

4. Verfahren nach Anspruch 1, **dadurch gekennzeichnet**, daß das Datenverarbeitungsprogramm eine dekodierung der deterministischen Muster durchführt.

5. Verfahren nach Anspruch 1, **dadurch gekennzeichnet**, daß das Datenverarbeitungssystem (4) als hochvernetztes neuronales Netz realisiert ist, das in Kombination mit einer Fuzzy-Kalkulationsmethode Informationsmuster des Biosensors (2) dekodiert.

6. Verfahren nach Anspruch 1, **dadurch gekennzeichnet**, daß aufgrund der erstellten Muster (Code) automatisch mittels des Stellgliedes (5) Maßnahmen vorgenommen werden, die das biologische System (1) verändern.

7. Verfahren nach Anspruch 1, **dadurch gekennzeichnet**, daß zum Trainieren des künstlichen neuronalen Netzes (4) ein Hormon, insbesondere ein Parathormon, verwendet wird, aus dem das Netzwerk den kodierten Determinismus des biologischen lebenden Systems (1) erlernt, wobei die verwendeten Biosensoren (2) mit einem Hormonrezeptorprotein, insbesondere mit einem Parathormon-Rezeptorprotein, versehen werden.

8. Verfahren nach Anspruch 1, **dadurch gekennzeichnet**, daß die Signale der verwendeten Biosensoren (2) in kurzen Zeitintervallen erfaßt und analysiert werden.

9. Dosiereinrichtung mit
- mindestens einem Biosensor (2), der in einem biologischen System (1) implantiert ist und dessen für einen bestimmten krankheitsfall symptomatischen Meßsignale einem trainierten neuronalen Netz (4) zugeführt werden; und
- einem Datenverarbeitungsprogramm zur Simulation des neuronalen Netzes, das aufgrund der Meßsignale raum-zeitliche Muster der verschiedenen, für einen bestimmten Krankheitsfall symptomatischen Meßgrößen erstellt;
**dadurch gekennzeichnet**, daß
- das Datenverarbeitungsprogramm die Änderungen der raum-zeitlichen Muster analysiert und den zukünftigen Verlauf der Meßgrößen mit Hilfe dieser Analyse vorausbestimmt; und
- Stellglieder (5) anhand der vorausbestimmten Werte der Meßgröße eingestellt werden.

10. Einrichtung nach Anspruch 9, **dadurch gekennzeichnet**, daß der Biosensor (2) kontinuierlich Konzentrationen von Substanzen, insbesondere von Hormonen, des biologischen Systems (1) mißt.

11. Einrichtung nach Anspruch 9, **dadurch gekennzeichnet**, daß das Datenverarbeitungssystem (4) ein hochvernetztes neuronales Netz ist, das in der Lage ist, Fuzzy-Kalkulationsmethoden durchzuführen.

12. Einrichtung nach Anspruch 9, **dadurch gekennzeichnet**, daß das Datenverarbeitungsprogramm die Klassifizierung von typischen deterministischen Mustern vornimmt.

13. Einrichtung nach Anspruch 9, **dadurch gekennzeichnet**, daß das Stellglied (5) eine computergesteuerte Applikationspumpe ist.

14. Einrichtung nach Anspruch 9, **dadurch gekennzeichnet**, daß der verwendete Biosensor (2) direkt an das neuronale Netzwerk (4) gekoppelt ist, wobei die Kopplung elektrisch ist.

15. Einrichtung nach Anspruch 9, **dadurch gekennzeichnet**, daß der Biosensor (2) direkt an ein neuronales Netzwerk (4) gekoppelt ist, wobei die Kopplung optisch ist.

16. Einrichtung nach Anspruch 9 und 14, **dadurch gekennzeichnet**, daß der Biosensor (2) ein Parathormon-Rezeptorprotein enthält.

17. Einrichtung nach Anspruch 9 und 14, **dadurch gekennzeichnet**, daß der Biosensor (2) aus gentechnologisch hergestellten Rezeptorproteinen für Hormone besteht.

18. Einrichtung nach Anspruch 9, **dadurch gekennzeichnet**, daß ein Biosensor aus einem Sensormolekül besteht, das ein gentechnologisch synthetisiertes Protein des Nebenschilddrüsen-Hormonrezeptors ist.

19. Einrichtung nach Anspruch 16, **dadurch gekennzeichnet**, daß das Rezeptorprotein ein 7-fach membrangängiges Protein ist.

20. Einrichtung nach Anspruch 16, **dadurch gekennzeichnet**, daß das Rezeptorprotein eine Modifikation der Proteinsequenz des 7-fach membrangängigen Rezeptorproteins ist.

## Claims

1. An automatic dosing method, consisting of the method steps:
- storing continuous measurement sequences of different measured values of a known biological system which are symptomatic of a specific case of illness;
- training an artificial neural network (4), which is simulated by a data processing program, with the aid of the measurement sequences provided by the known biological system;
- taking continuous measurement sequences which are symptomatic of a specified case of illness by means of at least one bio-sensor (2) which is implanted in the biological system and whose measurement signals are fed to the neural network;
and characterized by the following further method steps:
- analysing changes in the space-time pattern of the different measurement values produced by the artificial neural network (4);
- determining in advance the future course of the measured values with the aid of this analysis; and
- adjusting the control member (5) of a dosing device on the basis of the values of the measured values determined in advance.

2. A method according to claim 1, characterized in that the space-time patterns which are produced are associated with specific illness states.

3. A method according to claim 1, characterized in that an individual pattern analysis is associated with each individual living biological system, describing the state of the biological system.

4. A method according to claim 1, characterized in that the data processing program carries out a decoding of the deterministic patterns.

5. A method according to claim 1, characterized in that the data processing system (4) is realised as a highly-branched network which decodes information patterns of the bio-sensor (2) in conjunction with a fuzzy calculation method.

6. A method according to claim 1, characterized in that measures are taken automatically by means of the control member (5) on the basis of the patterns (code) which is produced, which measures alter the biological system (1).

7. A method according to claim 1, characterized in that a hormone, especially a parathyroid hormone, is used for training the artificial neural network (4), from which the network learns the coded determinism of the biological living system (1), wherein the bio-sensors (2) employed are provided with a hormone receptor protein, especially with a parathyroid hormone receptor protein.

8. A method according to claim 1, characterized in that the signals of the bio-sensors (2) which are employed are detected and analysed in short intervals of time.

9. Dosing apparatus with
- at least one bio-sensor (2), which is implanted in a biological system (1) and whose measurement signals symptomatic of a specific case of illness are fed to a trained neural network (4); and
- a data processing program for simulating the neural network, which produces space-time patterns on the basis of the measurement signals of the different measured values symptomatic of a specific case of illness;
characterized in that
- the data processing program analyses the changes in the space-time pattern and determines in advance the future course of the measured values with the aid of this analysis; and
- control members (5) are adjusted on the basis of the values of the measured values determined in advance.

10. Apparatus according to claim 9, characterized in that the bio-sensor (2) measures continuously concentrations of substances, especially hormones of the biological system (1).

11. Apparatus according to claim 9, characterized in that the data processing system (4) is a highly-branched neural network which is adapted to carry out fuzzy calculating methods.

12. Apparatus according to claim 9, characterized in that the data processing program carries out classification of typical deterministic patterns.

13. Apparatus according to claim 9, characterized in that the control member (5) is a computer-controlled administering pump.

14. Apparatus according to claim 9, characterized in that the bio-sensor (2) employed is coupled directly to the neural network (4), the coupling being electrical.

15. Apparatus according to claim 9, characterized in that the bio-sensor (2) is coupled directly to a neural network (4), the coupling being optical.

16. Apparatus according to claims 9 and 14, characterized in that the bio-sensor (2) contains a parathyroid hormone receptor protein.

17. Apparatus according to claims 9 and 14, characterized in that the bio-sensor (2) consists of receptor proteins for hormones manufactured by gene technology.

18. Apparatus according to claim 9, characterized in that a bio-sensor consists of a sensor molecule which is a protein of the parathyroid gland hormone receptor synthesised by gene technology.

19. Apparatus according to claim 16, characterized in that the receptor protein is a 7-fold membrane-passing protein.

20. Apparatus according to claim 16, characterized in that the receptor protein is a modification of the protein sequence of the 7-fold membrane-passing receptor protein.

## Revendications

1. Procédé de dosage automatique comprenant les étapes de procédé suivantes consistant :
- à mémoriser des séries de mesures en continu de différentes variables de mesure d'un système biologique connu, caractéristiques d'un certain type de maladie;
- à faire subir un apprentissage à un réseau neuronal artificiel (4) simulé par un programme de traitement de données, grâce aux séries de mesures effectuées sur le système biologique connu;
- à enregistrer en continu des séries de mesures caractéristiques d'un certain type de maladie au moins d'un biodétecteur (2) qui est implanté dans le système biologique et dont les signaux de mesure sont introduits dans le réseau neuronal;
et caractérisé en ce qu'il comprend les étapes de procédé supplémentaires suivantes consistant :
- à analyser les changements des profils spatio-temporels des différentes variables de mesure établis par le réseau neuronal artificiel (4),
- à prévoir, grâce à cette analyse, l'évolution future des variables de mesure; et
- à ajuster l'élément de réglage (5) d'un dispositif de dosage en fonction des valeurs prévues des variables de mesure.

2. Procédé conforme à la revendication 1, caractérisé en ce que les profils spatio-temporels établis sont attribués à certains états de maladie.

3. Procédé conforme à la revendication 1, caractérisé en ce que l'on attribue à chaque système biologique vivant individuel une analyse de profil individuelle décrivant l'état du système biologique.

4. Procédé conforme à la revendication 1, caractérisé en ce que le programme de traitement de données effectue un décodage des profils déterministes.

5. Procédé conforme à la revendication 1, caractérisé en ce que le système de traitement de données (4) est réalisé sous la forme d'un réseau neuronal fortement développé qui décode les profils d'informations du biodétecteur (2) à l'aide d'un procédé de calcul faisant appel à la logique floue.

6. Procédé conforme à la revendication 1, caractérisé en ce que des mesures visant à modifier le système biologique (1) sont prises automatiquement à partir des profils établis (code) et au moyen de l'élément de réglage (5).

7. Procédé conforme à la revendication 1, caractérisé en ce que l'on utilise pour l'apprentissage du réseau neuronal artificiel (4) une hormone, en particulier une parathormone, à l'aide de laquelle le réseau apprend le déterminisme codé du système biologique vivant (1), les biodétecteurs (2) utilisés étant pourvus d'une protéine récepteur d'hormones, en particulier d'une protéine récepteur de parathormone.

8. Procédé conforme à la revendication 1, caractérisé en ce que les signaux de mesure des biodétecteurs (2) utilisés sont enregistrés et analysés à des intervalles courts.

9. Dispositif de dosage comprenant
- au moins un biodétecteur (2) qui est implanté dans un système biologique (1) et qui émet des signaux de mesure caractéristiques d'un certain type de maladie, lesquels signaux sont introduits dans un réseau neuronal ayant subi un apprentissage (4), et
- un programme de traitement de données servant à la simulation du réseau neuronal, lequel programme établit, à partir des signaux de mesure, des profils spatio-temporels des différentes variables de mesure caractéristiques d'un certain type de maladie,
caractérisé en ce que le programme de traitement de données analyse les changements des profils spatio-temporels et, grâce à cette analyse, prévoit l'évolution future des variables de mesure, et en ce que des éléments de réglage (5) sont ajustés en fonction des valeurs des variables de mesure prévues.

10. Dispositif conforme à la revendication 9, caractérisé en ce que le biodétecteur (2) mesure en continu des concentrations de substances, en particulier d'hormones, dans le système biologique (1).

11. Dispositif conforme à la revendication 9, caractérisé en ce que le système de traitement de données (4) est un réseau neuronal fortement développé capable d'effectuer des procédés de calcul faisant appel à la logique floue.

12. Dispositif conforme à la revendication 9, caractérisé en ce que le programme de traitement de données effectue la classification de profils déterministes typiques.

13. Dispositif conforme à la revendication 9, caractérisé en ce que l'élément de réglage (5) est une pompe de distribution commandée par ordinateur.

14. Dispositif conforme à la revendication 9, caractérisé en ce que le biodétecteur (2) utilisé est couplé directement au réseau neuronal (4) par voie électrique.

15. Dispositif conforme à la revendication 9, caractérisé en ce que le biodétecteur (2) utilisé est couplé directement au réseau neuronal (4) par voie optique.

16. Dispositif conforme aux revendications 9 et 14, caractérisé en ce que le biodétecteur (2) contient une protéine récepteur de parathormone.

17. Dispositif conforme aux revendications 9 et 14, caractérisé en ce que le biodétecteur (2) est constitué de protéines récepteurs d'hormones préparées par génie génétique.

18. Dispositif conforme à la revendication 9, caractérisé en ce que le biodétecteur est constitué d'une molécule de détection qui est une protéine récepteur de l'hormone parathyroïdienne, synthétisée par génie génétique.

19. Dispositif conforme à la revendication 16, caractérisé en ce que la protéine récepteur est une protéine traversant 7 fois la membrane.

20. Dispositif conforme à la revendication 16, caractérisé en ce que la protéine récepteur est une variante de la protéine récepteur traversant 7 fois la membrane dont la séquence protéique a été modifiée.
